# EUROPÄISCHE PATENTSCHRIFT

(11) **EP 2 473 467 B1**
(45) Veröffentlichungstag und Bekanntmachung des Hinweises auf die Patenterteilung: **25.09.2013**
(21) Anmeldenummer: 11752179.9
(22) Anmeldetag: 26.08.2011
(51) Int. Cl.: C07C 51/21, C07C 51/215

(54) **VERFAHREN ZUR KATALYTISCHEN ERZEUGUNG VON AMEISENSÄURE**
METHOD FOR CATALYTICALLY PRODUCING FORMIC ACID
PROCÉDÉ POUR LA PRÉPARATION CATALYTIQUE D'ACIDE FORMIQUE

(30) Priorität: 08.06.2011 DE 102011077232; 17.09.2010 DE 102010045863
(43) Veröffentlichungstag der Anmeldung: 11.07.2012
(73) Patentinhaber: Jbach GmbH, 96120 Bischberg (DE)
(72) Erfinder: BÖSMANN, Andreas, 91093 Hessdorf (DE); WÖLFEL, René, 91336 Heroldsbach (DE); WASSERSCHEID, Peter, 91054 Erlangen (DE); TACCARDI, Nicola, 90427 Boxdorf-Nürnberg (DE); ALBERT, Jacob, 91056 Erlangen (DE)
(74) Vertreter: Ehnis, Tobias
(86) Internationale Anmeldenummer: PCT/EP2011/064749
(87) Internationale Veröffentlichungsnummer: WO 2012/034839

(56) Entgegenhaltungen:
- LOURDES CALVO ET AL.: "Formation of organic acids during the hydrolysis and oxidation of several wastes in sub- and supercritical water", IND. ENG. CHEM. RES., Bd. 41, 2002, Seiten 6503-6509, XP002666550,

## Beschreibung

Die Erfindung betrifft ein Verfahren zur katalytischen Erzeugung von Ameisensäure.

Bisher wird Ameisensäure vor allem durch Carbonylierung von Methanol mittels Kohlenmonoxid und anschließender Hydrolyse des gebildeten Methylformiats hergestellt. Das bei der Hydrolyse freigesetzte Methanol wird in den ersten Prozessschritt zurückgeführt. Die bei dem Verfahren eingesetzten Ausgangsstoffe Methanol und Kohlenmonoxid werden üblicherweise aus Synthesegas gewonnen. Synthesegas wird im Allgemeinen aus Erdgas, Kohle oder Erdölfraktionen hergestellt. Bei der Herstellung des Methanols für die Ameisensäureherstellung aus Synthesegas und bei der Carbonylierung des Methanols werden Katalysatoren eingesetzt, die als Zentralatom ausschließlich Edelmetalle aufweisen.

Aus Jin, F. und Enomoto, H., BioResources 4(2), 2009, Seiten 704-713 ist es bekannt, durch hydrothermale Umwandlung aus Reisschalen, Zellulosestärke und Glucose Essigsäure mit einer Reinheit zwischen 68,5 und 90% herzustellen, wobei der nicht aus Essigsäure bestehende Anteil zum größten Teil aus Ameisensäure besteht. Bei dem Verfahren wird Wasserstoffperoxid als Oxidationsmittel bei einer Temperatur von 300 °C und erhöhtem Druck eingesetzt. Nachteilig bei diesem Verfahren ist, dass Wasserstoffperoxid verhältnismäßig teuer ist.

Aus Niemelä, K., Biomass 15 (1988), Seiten 223-231 ist es bekannt, Zellulose mit Natriumhydroxid bei 170 bis 190 °C unter einem Sauerstoffdruck von 200 bis 400 kPa in Carbonsäuren zu verwandeln. Unter den dabei entstehenden Carbonsäuren befindet sich auch Ameisensäure.

Aus Khenkin, A. M. und Neumann, R., Adv. Synth. Catal. 2002, 344, Nr. 9, Seiten 1017-1021 ist die durch [IMo₆O₂₄]⁵⁻ katalysierte aerobe Oxidation vicinaler Diole bekannt.

Aus US 5,695,606 ist ein Verfahren zur oxidativen Delignifizierung von Holzmasse und Holzfasern bekannt. Bei der oxidativen Delignifizierung soll selektiv Lignin oxidiert werden und Zellulose erhalten bleiben. Dabei werden die Holzmasse und Holzfasern mit einem Polyoxometallat, wie beispielsweise H₅[PV₂Mo₁₀O₄₀], unter erhöhtem Druck und erhöhter Temperatur in Kontakt gebracht. Dabei wird das Polyoxometallat reduziert. Das Verfahren kann eine Reoxidierung des Polyoxometallats umfassen.

Aus Khenkin, A. M. and Neumann, R., J. Am. Chem. Soc. 2008, 130, 14474 - 14476 ist die oxidative Spaltung von C-C-Bindungen primärer Alkohole und vicinaler Diole, wie 1,2-Ethandiol, bekannt. Als Katalysator wird dabei H₅PV₂Mo₁₀O₄₀ verwendet.

Aus Khenkin, A. M., et al., Journal of Catalysis 182, 82 - 91 (1999) ist die Reaktion von Aldehyden mit H₅PV₂Mo₁₀O₄₀-Poly-oxometallaten bekannt. Bei dem Aldehyd kann es sich beispielsweise um Isobutyraldehyd handeln.

Aus Lourdes Calvo und David Vallejo, Ind. Eng. Chem. Res., Band 41, 2002, Seiten 6503 - 6509 ist ein Verfahren zur Herstellung organischen Säuren, beispielsweise Ameisensäure, durch Hydrolyse und Oxidation von organischen Abfallströmen in superkritischem Wasser bekannt.

Aufgabe der vorliegenden Erfindung ist es, ein alternatives Verfahren zur Herstellung von Ameisensäure anzugeben.

Die Erfindung wird durch die Merkmale des Anspruchs 1 gelöst. Zweckmäßige Ausgestaltungen ergeben sich aus den Merkmalen der Ansprüche 2 bis 11.

Erfindungsgemäß ist ein Verfahren zur katalytischen Erzeugung von Ameisensäure vorgesehen, wobei ein als Katalysator dienendes Polyoxometallat-Ion der allgemeinen Formel [PMoₓVyO₄₀]⁻⁵ bei einer Temperatur unterhalb von 120 °C mit einem alpha-Hydroxyaldehyd, einer alpha-Hydroxycarbonsäure, einem Kohlenhydrat oder einem Glycosid in einer flüssigen Lösung in Kontakt gebracht wird, wobei 6 < x < 11 und 1 < y < 6 und x + y = 12, wobei x und y jeweils eine ganze Zahl ist. Vorzugsweise wird dabei der bei dem Verfahren reduzierte Katalysator durch Oxidation wieder in seinen Ausgangszustand versetzt. Dadurch kann das Verfahren in einem kontinuierlichen Prozess durchgeführt werden. Im Sinne der Erfindung wird unter einem Katalysator auch ein Stoff verstanden, der bei dem Verfahren durch Oxidation verändert wird, wenn er nach dem Verfahren durch Reduktion wieder in seinen Ausgangszustand versetzt werden kann. Die erfindungsgemäße Erzeugung von Ameisensäure ist in diesem Sinne eine katalytische Erzeugung von Ameisensäure.

Die Lösung kann ein Lösemittel umfassen. Das Vorsehen eines Lösemittels ist nicht erforderlich, wenn das alpha-Hydroxyaldehyd, die alpha-Hydroxycarbonsäure, das Kohlenhydrat oder das Glycosid bereits in flüssiger Form vorliegt. Unter einem alpha-Hydroxyaldehyd wird jedes Molekül verstanden, bei welchem eine OH-Gruppe direkt an einem C-Atom gebunden ist, wobei an dem C-Atom auch direkt das C-Atom einer Aldehyd-Gruppe gebunden ist. Unter einer alpha-Hydroxycarbonsäure wird jedes Molekül verstanden, bei welchem eine OH-Gruppe direkt an einem C-Atom gebunden ist, wobei an dem C-Atom auch direkt das C-Atom einer Carboxy-Gruppe gebunden ist. Weiterhin werden unter alpha-Hydroxyaldehyden und alpha-Hydroxycarbonsäuren sämtliche Substanzen verstanden, welche ein alpha-Hydroxyaldehyd oder eine alpha-Hydroxycarbonsäure enthalten.

Bei der oxidativen katalytischen Erzeugung von Ameisensäure aus einem alpha-Hydroxyaldehyd oder einer alpha-Hydroxycarbonsäure muss stets die C-C-Bindung zwischen der Aldehyd-Gruppe oder der Carboxy-Gruppe und dem die OH-Gruppe aufweisenden C-Atom gespalten werden. Eine derartige oxidative katalytische Spaltung ist bisher nicht bekannt. Auch Hinweise auf eine oxidative katalytische Spaltung einer C-C-Bindung zwischen dem C-Atom einer Aldehyd-Gruppe oder Carboxy-Gruppe und einem eine OH-Gruppe aufweisenden C-Atom, die zur Bildung von Ameisensäure aus einem alpha-Hydroxyaldehyd oder einer alpha-Hydroxycarbonsäure erforderlich ist, sind dem Stand der Technik nicht zu entnehmen.

Eine derartige katalytische Spaltung erfolgt auch bei der Erzeugung von Ameisensäure aus Kohlenhydraten oder einem Glycosid. Bei der oxidativen Spaltung benachbarter C-Atome eines Kohlenhydrats oder des Zuckerbestandteils des Glycosids, wobei an zumindest einem der C-Atome eine OH-Gruppe gebunden ist, entsteht immer ein alpha-Hydroxyaldehyd oder eine alpha-Hydroxycarbonsäure. Die darin enthaltene C-C-Bindung zur Aldehyd- oder Carboxy-Gruppe muss zur Erzeugung von Ameisensäure stets gespalten werden.

Das erfindungsgemäße Verfahren hat den Vorteil, dass es auf wirtschaftliche Weise der Erzeugung von Ameisensäure aus einem nachwachsenden Rohstoff erlaubt. Die knapper werdenden fossilen Brennstoffe müssen dazu nicht eingesetzt werden. Es ist auch nicht erforderlich, aus nachwachsenden Rohstoffen zunächst Synthesegas zu erzeugen. Dies wäre mit einem erheblichen Aufwand verbunden, da derartige Rohstoffe Stickstoff und/oder Schwefel enthaltende Verbindungen enthalten, welche geeignete Edelmetalle als Zentralatom aufweisende Katalysatoren rasch deaktivieren würden. Derartige Verbindungen müssten daher vor der Erzeugung von Synthesegas aus nachwachsenden Rohstoffen aufwendig entfernt werden.

Alpha-Hydroxyaldehyde, Kohlenhydrate und Glycoside kommen dagegen in einer großen Zahl der nachwachsenden Rohstoffe, wie beispielsweise Stärke, Zellulose oder Hemizellulose vor. Stärke, Zellulose und Hemizellulose fallen in großen Mengen als Produkt aus Ackerpflanzen oder beim industriellen Holzaufschluss, beispielsweise zur Papierherstellung an.

Das erfindungsgemäße Verfahren zeichnet sich u. a. dadurch aus, dass die Ameisensäure auf direktem Wege aus alpha-Hydroxyaldehyd, alpha-Hydroxycarbonsäure, Kohlenhydrat oder Glycosid enthaltenden Rohstoffen oder Reststoffen mit verhältnismäßig hoher Selektivität erzeugt werden kann. Die Selektivität kann zwischen 50 und 99 % liegen. Insbesondere hat es sich gezeigt, dass beim erfindungsgemäßen Verfahren beim Einsatz von Oligosacchariden oder Polysacchariden als Kohlenhydrat mit hoher Selektivität eine katalytische Umsetzung erfolgt, ohne dass vorher eine Hydrolyse zu Monosacchariden erfolgen muss. Unter Selektivität wird das Verhältnis der Molzahl der C-Atome, die insgesamt in der bei dem Verfahren erzeugten Ameisensäure enthalten sind zu der Molzahl der C-Atome, die insgesamt in dem bei dem Verfahren eingesetzten Ausgangsstoff alpha-Hydroxyaldehyd, alpha-Hydroxycarbonsäure, Kohlenhydrat oder Glycosid enthalten sind, verstanden. Selektivität ist demnach n(HCOOH)/n(C-Atome im Ausgangsstoff). Die Oxidation des reduzierten Katalysators kann gleichzeitig mit der katalytischen Erzeugung von Ameisensäure oder in einem separaten Verfahrensschritt und insbesondere auch räumlich getrennt von der Oxidation des alpha-Hydroxyaldehyds, der alpha-Hydroxycarbonsäure, des Kohlenhydrats oder des Glycosids erfolgen. Dadurch können sowohl für die Oxidation des alpha-Hydroxyaldehyds, der alpha-Hydroxycarbonsäure, des Kohlenhydrats oder des Glycosids als auch für die Reoxidation des Katalysators jeweils optimale Bedingungen, z. B. hinsichtlich Druck und Temperatur, gewählt werden. Weiterhin kann dabei eine möglicherweise erfolgende Dehydratation von alpha-Hydroxyaldehyd enthaltenden Molekülen vermindert werden. Die beim erfindungsgemäßen Verfahren entstehende Prozesswärme kann von einem anderen Prozess, insbesondere einem Prozess, bei dem die gewonnene Ameisensäure umgesetzt wird, genutzt werden. Weiterhin kann die bei dem Verfahren entstehende Ameisensäure direkt mittels eines heterogenen Katalysators, wie z. B. Platin oder Palladium, in Wasserstoff und Kohlendioxid umgesetzt werden.

Bei dem alpha-Hydroxyaldehyd, der alpha-Hydroxycarbonsäure, dem Kohlenhydrat oder dem Glycosid kann es sich um ein Monosaccharid, insbesondere einer Aldose, Disaccharid, Oligosaccharid oder Polysaccharid, Stärke, Zellulose, Hemizellulose, Glucose, Saccharose, Xylose, Zellobiose, Xylan, ein Hetereooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoffen oder, insbesondere nachwachsenden, insbesondere unbehandelten, Rohstoff handeln. Unbehandelt bedeutet dabei, dass dieser zuvor nicht chemisch aufgeschlossen worden ist. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein.

Viele der genannten Rohstoffe fallen als Nebenprodukte, beispielsweise bei der Papierherstellung oder Holzverarbeitung an. Sie stehen damit als günstiger Ausgangsstoff für das erfindungsgemäße Verfahren zur Verfügung. Das erfindungsgemäße Verfahren kann dadurch sehr kostengünstig durchgeführt werden. Dazu trägt auch die Oxidation des bei der katalysierten Reaktion reduzierten Katalysators (= Reoxidation) bei, die eine sehr lange Verwendung des Katalysators erlaubt.

Bei einer Ausgestaltung des Verfahrens erfolgt das Oxidieren des Katalysators durch Einblasen von Sauerstoff oder eines, insbesondere mehr als 10 Vol.-%, Sauerstoff enthaltenden Gasgemischs in die Lösung oder durch Zusatz eines Oxidationsmittels, insbesondere H₂O₂ oder N₂O, zu der Lösung. Bei dem Gasgemisch kann es sich beispielsweise um Luft oder synthetische Luft handeln.

Die Lösung kann ein polares Lösemittel, insbesondere Wasser, Ameisensäure, eine ionische Flüssigkeit oder Mischungen dieser Lösemittel umfassen.

Als vorteilhaft hat es sich erwiesen, wenn der Lösung eine anorganische Säure, insbesondere Schwefelsäure, Phosphorsäure, Salzsäure oder Kohlensäure, eine, insbesondere 2 bis 20 C-Atome aufweisende, Carbonsäure oder eine, insbesondere 2 bis 20 C-Atome aufweisende, Carbondisäure zugesetzt wird. Dadurch kann eine Depolymerisation des alpha-Hydroxyaldehyds, der alpha-Hydroxycarbonsäure, des Kohlenhydrats, des Glycosids oder der genannten Rohstoffe oder Reststoffe beschleunigt werden.

Weiterhin kann der Lösung ein Tensid oder ein sonstiges Additiv, insbesondere eine Sulfonsäure, insbesondere Methansulfonsäure, Trifluormethansulfonsäure, Camphersulfonsäure, Toluolsulfonsäure, insbesondere para-Toluolsulfonsäure, Chlorsulfonsäure, Xylolsulfonsäure, Benzolsulfonsäure oder ein Derivat einer der genannten Säuren, insbesondere Chlorbenzolsulfonsäure, insbesondere para-Chlorbenzolsulfonsäure, oder ein Salz einer der genannten Säuren oder des Derivats oder ein sonstiger Stoff, der in einer wässrigen Lösung eine der genannten Säuren oder das Derivat bildet, zugesetzt werden. Dadurch können insbesondere die Reststoffe, insbesondere Altpapier, und die nachwachsenden Rohstoffe, insbesondere Holz, insbesondere in Form von Holzmehl sowie Bakterien, insbesondere Cyanobakterien, besser umgesetzt werden. Insgesamt kann dadurch eine bessere Ausbeute erzielt werden. Para-Chlorbenzolsulfonsäure hat den Vorteil, dass deren Zusatz keine Schaumbildung bewirkt.

Das Inkontaktbringen erfolgt vorzugsweise bei einer Temperatur von 15 bis 120 °C, insbesondere 50 bis 110 °C, insbesondere 60 bis 100 °C, insbesondere 70 bis 95 °C, insbesondere 85 bis 95 °C. Je höher die Temperatur ist, desto schneller erfolgt die katalytische Umsetzung. Bei Temperaturen bis zu 100 °C erfolgt die Umsetzung mit hoher Selektivität. Je mehr die Temperatur 100 °C übersteigt, desto mehr nimmt die Selektivität der Umsetzung ab. Ein Inkontaktbringen bei einer Temperatur von höchstens 100 °C ist daher vorteilhaft.

Als vorteilhaft hat es sich weiterhin erwiesen, wenn das Inkontaktbringen unter einem Sauerstoffpartialdruck von 1 bis 500 bar, insbesondere 5 bis 150 bar, insbesondere 20 bis 120 bar, insbesondere 30 bis 80 bar, insbesondere 50 bis 75 bar, erfolgt. Je höher der Sauerstoffpartialdruck ist, desto schneller erfolgt die Oxidation des bei dem Verfahren reduzierten Katalysators.

Bei dem Verfahren erzeugte Ameisensäure kann durch Destillation, Reaktivdestillation, Extraktion, insbesondere zusammen mit dem Katalysator, insbesondere durch Zugabe einer Base, insbesondere einem Amin, durch Strippen oder nach Umsetzung mit einem heterogenen Katalysator, insbesondere Platin oder Palladium, als bei der Umsetzung entstandene Reaktionsprodukte Wasserstoff und Kohlendioxid aus der Lösung entfernt werden. Der heterogene Katalysator kann dabei beispielsweise in Form eines Drahtnetzes oder einer porösen schwammartigen Struktur vorliegen. Sofern bei der katalytischen Erzeugung von Ameisensäure gleichzeitig eine Oxidation des Katalysators erfolgt, sollte wegen der Reaktivität des Wasserstoffs die Umsetzung mit dem heterogenen Katalysator nicht gleichzeitig mit der Oxidation des Katalysators erfolgen, um beispielsweise eine Knallgasreaktion zu vermeiden. Bei der Extraktion kann es sich auch um eine Reaktivextraktion handeln.

Vorzugsweise wird der Lösung zur Extraktion der Ameisensäure als Extraktionsmittel ein Ether, insbesondere Di-isopropylether, Ethyl-tert.-butylether, Ethyl-iso-butylether, Ethyl-sec.-butylether, Methyl-tert.-pentylether, Di-n-propylether, Ethyl-n-butylether, Ethyl-tert.-pentylether, Diisobutylether oder Di-n-butylether, zugesetzt. Zur Extraktion der Ameisensäure zusammen mit dem Katalysator kann der Lösung ein Amid, insbesondere N,N-Di-n-butylformamid, N-Di-n-acetamid, N-methyl-N-heptylformamid, N-n-butyl-N-2-ethylhexylformamid oder N-n-butyl-N-cyclohexylformamid, zugesetzt werden. Die Extraktion zusammen mit dem Katalysator ist insbesondere dann vorteilhaft, wenn die Lösung das alpha-Hydroxyaldehyd, die alpha-Hydroxycarbonsäure, das Kohlenhydrat oder das Glycosid nur in niedriger Konzentration enthält. Das kann insbesondere bei nachwachsenden Rohstoffen, wie z.B. Algen, von denen oft nur wenige Gewichtsprozent in Wasser vorhanden sind, der Fall sein.

Nachfolgend wird die Erfindung anhand von Ausführungsbeispielen näher erläutert.

### Beispiel 1:

### Oxidation von Glucose in Wasser bei 80 °C

Zu 100 g destilliertem Wasser werden bei Raumtemperatur 3,30 g Glucose und 1,74 g H₅PMo₁₀V₂O₄₀ zugesetzt. In einem Rührkessel-Autoklaven mit Gaseintragsrüher wird die Mischung unter 30 bar Sauerstoff auf 80 °C erwärmt und für 7 h gerührt. Nach Abkühlen und Entspannen entspricht der Ameisensäuregehalt in der Lösung einer Ausbeute von 51 %. Andere Stoffe sind mittels NMR in der Reaktionslösung nicht mehr nachweisbar.

### Beispiel 2:

### Oxidation von Glucose in Wasser bei 70 °C

Die Reaktion wird wie für Beispiel 1 beschrieben durchgeführt. Abweichend davon erfolgt die Reaktion für 22 h bei 70 °C. Der Ameisensäuregehalt in der Lösung entspricht einer Ausbeute von 51 %. Andere Stoffe sind mittels NMR in der Reaktionslösung nicht mehr nachweisbar.

### Beispiel 3:

### Oxidation von Glucose in Wasser bei 90 °C

Die Reaktion wird wie für Beispiel 1 beschrieben durchgeführt. Abweichend davon erfolgt die Reaktion für 3 h bei 90 °C. Der Ameisensäuregehalt in der Lösung entspricht einer Ausbeute von 52 %. Andere Stoffe sind mittels NMR in der Reaktionslösung nicht mehr nachweisbar.

### Beispiel 4:

### Produktstabilität unter Reaktionsbedingungen

100 g destilliertem Wasser werden bei Raumtemperatur 4,70 g Ameisensäure zugesetzt. Zu dieser Lösung werden 1,74 g H₅PMo₁₀V₂O₄₀ gegeben. In einem Rührkessel-Autoklaven mit Gaseintragsrührer wird die Mischung unter 30 bar Sauerstoff auf 90 °C erwärmt und für 20 h gerührt. Nach Abkühlen und Entspannen sind in der Reaktionslösung 4,70 g Ameisensäure erhalten.

### Beispiel 5:

### Oxidation von Xylose

Die Reaktion wird wie für Beispiel 1 beschrieben durchgeführt. Statt der Glucose werden 3,30 g Xylose zugesetzt. Der Ameisensäuregehalt in der Lösung entspricht einer Ausbeute von 54 %. Andere Stoffe sind mittels NMR in der Lösung nicht mehr nachweisbar.

### Beispiel 6:

### Oxidation der Hemizellulose Xylan

Die Reaktion wird wie für Beispiel 1 beschrieben durchgeführt. Statt der Glucose werden 3,30 g Xylan zugesetzt. Der Ameisensäuregehalt in der Lösung entspricht einer Ausbeute von 33 %.

### Beispiel 7:

### Oxidation von Zellulose

Die Reaktion wird wie für Beispiel 1 beschrieben durchgeführt. Statt der Glucose werden 3,30 g Zellulose zugesetzt und in dem Wasser suspendiert. Die Reaktionszeit beträgt abweichend von Beispiel 1 97 Stunden. Der Ameisensäuregehalt in der Lösung entspricht einer Ausbeute von 27 %.

### Beispiel 8:

### Oxidation von Saccharose

Die Reaktion wird wie für Beispiel 1 beschrieben durchgeführt. Statt der Glucose werden 3,30 g Saccharose zugesetzt. Der Ameisensäuregehalt in der Lösung entspricht einer Ausbeute von 48 %. Andere Stoffe sind mittels NMR in der Lösung nicht mehr nachweisbar.

### Beispiel 9:

### Oxidation von Pappelholz

Die Reaktion wird wie für Beispiel 1 beschrieben durchgeführt. Statt der Glucose werden 3,30 g Pappelholz in Form eines Sägemehls zugesetzt und in dem Wasser suspendiert. Der Ameisensäuregehalt in der Lösung entspricht nach 97 h Reaktionszeit, bezogen auf die eingesetzte Masse, einer Ausbeute von 19 %.

### Beispiel 10:

### Oxidation von Pappelholz mit Zusatz eines Additivs

Die Reaktion wird wie für Beispiel 1 beschrieben durchgeführt. Statt der Glucose werden 2,70 g Pappelholz in Form eines Sägemehls zugesetzt und in dem Wasser suspendiert. Weiterhin werden 1,91 g para-Toluolsulfonsäure als Additiv zugesetzt. Der Ameisensäuregehalt in der Lösung entspricht nach 24 h Reaktionszeit, bezogen auf die eingesetzte Masse, einer Ausbeute von 39 %.

### Beispiel 11:

### Oxidation mit erhöhter Katalysatormenge

Die Reaktion wird wie für Beispiel 1 beschrieben durchgeführt. Abweichend davon werden 7,40 g H₅PMo₁₀V₂O₄₀ anstelle von 1,74 g H₅PMo₁₀V₂O₄₀ zugesetzt. Der Ameisensäuregehalt in der Lösung entspricht einer Ausbeute von 52 %. Andere Stoffe sind mittels NMR in der Lösung nicht mehr nachweisbar.

### Beispiel 12:

### Oxidation mit erhöhter Menge an Substrat

Die Reaktion wird wie für Beispiel 1 beschrieben durchgeführt. Abweichend davon werden 10,0 g Glucose anstelle von 3,30 g Glucose zugesetzt. Die Reaktionszeit beträgt 51 h statt 7 h. Der Ameisensäuregehalt in der Lösung entspricht einer Ausbeute von 49 %. Andere Stoffe sind mittels NMR in der Lösung nicht mehr nachweisbar.

### Beispiel 13:

### Oxidation mit erhöhter Katalysatormenge und erhöhter Substratmenge

Die Reaktion wird wie für Beispiel 1 beschrieben durchgeführt. Abweichend davon werden 7,40 g H₅PMo₁₀V₂O₄₀ und 10,0 g Glucose zugesetzt. Die Reaktionszeit beträgt 24 h statt 7 h. Der Ameisensäuregehalt in der Lösung entspricht einer Ausbeute von 53 %. Andere Stoffe sind mittels NMR in der Lösung nicht mehr nachweisbar.

### Beispiel 14:

### Oxidation bei vermindertem Druck

Die Reaktion wird wie für Beispiel 1 beschrieben durchgeführt. Abweichend davon erfolgt die Oxidationsreaktion von Glucose bei 10 bar Sauerstoff anstatt bei 30 bar Sauerstoff. Der Ameisensäuregehalt in der Lösung entspricht einer Ausbeute von 49 %. Andere Stoffe sind mittels NMR in der Lösung nicht mehr nachweisbar.

### Beispiel 15:

### Oxidation bei erhöhtem Druck

Die Reaktion wird wie für Beispiel 1 beschrieben durchgeführt. Abweichend davon erfolgt die Oxidationsreaktion von Glucose bei 80 bar Sauerstoff anstatt bei 30 bar Sauerstoff. Der Ameisensäuregehalt in der Lösung entspricht einer Ausbeute von 51 %. Andere Stoffe sind mittels NMR in der Lösung nicht mehr nachweisbar.

### Beispiel 16:

### Oxidation mit Luft

Die Reaktion wird wie für Beispiel 1 beschrieben durchgeführt. Abweichend davon erfolgt die Oxidationsreaktion von Glucose bei 80 bar Luft anstatt bei 30 bar Sauerstoff. Der Ameisensäuregehalt in der Lösung entspricht einer Ausbeute von 51 %. Andere Stoffe sind mittels NMR in der Lösung nicht mehr nachweisbar.

### Beispiel 17:

### Oxidation mit einem sauerstoffhaltigen Gasgemisch

Die Reaktion wird wie für Beispiel 1 beschrieben durchgeführt. Abweichend davon erfolgt die Oxidationsreaktion von Glucose anstatt bei 30 bar Sauerstoff bei 60 bar einer Mischung aus gleichen Volumenanteilen Sauerstoff und Stickstoff. Der Ameisensäuregehalt in der Lösung entspricht einer Ausbeute von 53 %. Andere Stoffe sind mittels NMR in der Lösung nicht mehr nachweisbar.

### Beispiel 18:

### Extraktion von Ameisensäure

Zu einer einer Reaktionslösung entsprechenden Lösung aus 10,0 g Wasser, 0,174 g H₅PMo₁₀V₂O₄₀ und 5,0 g Ameisensäure werden 5,0 g Di-n-butylether als Extraktionsmittel zugesetzt und mit der wässrigen Phase gemischt. Der orange gefärbte Katalysator verbleibt dabei nach optischer Beurteilung vollständig in der wässrigen Phase während die Ameisensäure in die organische Phase übergeht. Als Verteilungskoeffizient der Ameisensäure wird mittels NMR ein Wert von 0,81 bestimmt.

### Beispiel 19:

### Extraktion von Ameisensäure und Katalysator

Zu einer einer Reaktionslösung entsprechenden Lösung aus 10,0 g Wasser, 0,174 g H₅PMo₁₀V₂O₄₀ und 5,0 g Ameisensäure werden 5,0 g N,N-Di-n-butylformamid als Extraktionsmittel zugesetzt und mit der wässrigen Phase gemischt. Der orange gefärbte Katalysator geht dabei nach optischer Beurteilung vollständig in die organische Phase über. Als Verteilungskoeffizient der Ameisensäure wird mittels NMR ein Wert von 1,27 bestimmt.

## Patentansprüche

1. Verfahren zur katalytischen Erzeugung von Ameisensäure, wobei ein als Katalysator dienendes Polyoxometallat-Ion der allgemeinen Formel [PMoₓV_{y}O₄₀]⁵⁻ bei einer Temperatur unterhalb von 120 °C mit einem alpha-Hydroxyaldehyd, einer alpha-Hydroxycarbonsäure, einem Kohlenhydrat oder einem Glycosid in einer flüssigen Lösung in Kontakt gebracht wird, wobei 6 < x < 11 und 1 < y < 6 und x + y = 12, wobei x und y jeweils eine ganze Zahl ist.

2. Verfahren nach Anspruch 1, wobei der bei dem Verfahren reduzierte Katalysator durch Oxidation wieder in seinen Ausgangszustand versetzt wird.

3. Verfahren nach einem der vorhergehenden Ansprüche, wobei das alpha-Hydroxyaldehyd, die alpha-Hydroxycarbonsäure, das Kohlenhydrat oder das Glycosid ein Monosaccharid, insbesondere eine Aldose, Disaccharid, Oligosaccharid oder Polysaccharid, Stärke, Zellulose, Hemizellulose, Glucose, Saccharose, Xylose, Zellobiose, Xylan, ein Heterooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoff oder, insbesondere nachwachsenden, Rohstoff, insbesondere eine Pflanze, ein Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage, oder ein Gemisch aus mindestens zwei der genannten Substanzen umfasst oder aus mindestens einer der genannten Substanzen oder dem Gemisch entstanden ist.

4. Verfahren nach einem der vorhergehenden Ansprüche, wobei das Oxidieren des Katalysators durch Einblasen von Sauerstoff oder eines, insbesondere mehr als 10 Volumenprozent, Sauerstoff enthaltenden Gasgemischs in die Lösung oder durch Zusatz eines Oxidationsmittels, insbesondere H₂O₂ oder N₂O, zu der Lösung erfolgt.

5. Verfahren nach einem der vorhergehenden Ansprüche, wobei die Lösung ein polares Lösemittel, insbesondere Wasser, Ameisensäure, eine ionische Flüssigkeit oder Mischungen dieser Lösemittel umfasst.

6. Verfahren nach einem der vorhergehenden Ansprüche, wobei der Lösung eine anorganische Säure, insbesondere Schwefelsäure, Phosphorsäure, Salzsäure oder Kohlensäure, eine, insbesondere 2 bis 20 C-Atome aufweisende, Carbonsäure oder eine, insbesondere 2 bis 20 C-Atome aufweisende, Carbondisäure zugesetzt wird.

7. Verfahren nach einem der vorhergehenden Ansprüche, wobei der Lösung ein Tensid oder ein sonstiges Additiv, insbesondere eine Sulfonsäure, insbesondere Methansulfonsäure, Trifluormethansulfonsäure, Camphersulfonsäure, Toluolsulfonsäure, insbesondere para-Toluolsulfonsäure, Chlorsulfonsäure, Xylolsulfonsäure, Benzolsulfonsäure oder ein Derivat einer der genannten Säuren, insbesondere Chlorbenzolsulfonsäure, insbesondere para-Chlorbenzolsulfonsäure, oder ein Salz einer der genannten Säuren oder des Derivats oder ein sonstiger Stoff, der in einer wässrigen Lösung eine der genannten Säuren oder das Derivat bildet, zugesetzt wird.

8. Verfahren nach einem der vorhergehenden Ansprüche, wobei das Inkontaktbringen bei einer Temperatur von 15 bis 120 °C, insbesondere 50 bis 110 °C, insbesondere 60 bis 100 °C, insbesondere 70 bis 95 °C, insbesondere 85 bis 95 °C, erfolgt.

9. Verfahren nach einem der vorhergehenden Ansprüche, wobei das Inkontaktbringen unter einem Sauerstoffpartialdruck von 1 bis 500 bar, insbesondere 5 bis 150 bar, insbesondere 20 bis 120 bar, insbesondere 30 bis 80 bar, insbesondere 50 bis 75 bar, erfolgt.

10. Verfahren nach einem der vorhergehenden Ansprüche, wobei die bei dem Verfahren erzeugte Ameisensäure durch Destillation, Reaktivdestillation, Extraktion, insbesondere zusammen mit dem Katalysator, insbesondere durch Umsetzen mit einer Base, insbesondere einem Amin, durch Strippen oder nach Umsetzung mit einem heterogenen Katalysator, insbesondere Platin oder Palladium, als dabei entstandene Reaktionsprodukte Wasserstoff und Kohlendioxid aus der Lösung entfernt wird.

11. Verfahren nach Anspruch 10, wobei der Lösung zur Extraktion der Ameisensäure als Extraktionsmittel ein Ether, insbesondere Di-isopropylether, Ethyl-tert.-butylether, Ethyl-iso-butylether, Ethyl-sec.-butylether, Methyl-tert.-pentylether, Di-n-propylether, Ethyl-n-butylether, Ethyl-tert.-pentylether, Diisobutylether oder Di-n-butylether, oder zur Extraktion der Ameisensäure zusammen mit dem Katalysator ein Amid, insbesondere N,N-Di-n-butylformamid, N-Di-n-acetamid, N-methyl-N-heptylformamid, N-n-butyl-N-2-ethylhexylformamid oder N-n-butyl-N-cyclohexylformamid, zugesetzt wird.

## Claims

1. A method for catalytically producing formic acid, wherein a polyoxometallate ion serving as catalyst and of the general formula [PMoₓV_{y}O₄₀]⁵⁻ is contacted at a temperature below 120°C with an alpha-hydroxyaldehyde, an alpha-hydroxycarboxylic acid, a carbohydrate, or a glycoside in a liquid solution, where 6 < x < 11 and 1 < y < 6, and x + y = 12, with x and y each being an integer.

2. The method of claim 1, wherein the catalyst reduced during the method is returned to its original state by oxidation.

3. The method of either of the preceding claims, wherein the alpha-hydroxyaldehyde, the alpha-hydroxycarboxylic acid, the carbohydrate, or the glycoside comprises a monosaccharide, more particularly an aldose, a disaccharide, oligosaccharide, or polysaccharide, starch, cellulose, hemicellulose, glucose, sucrose, xylose, cellobiose, xylan, a heterooligosaccharide, a heteropolysaccharide, glycolic acid, or lactic acid, or a raw material, more particularly renewable raw material, or residual substance comprising the alpha-hydroxyaldehyde, the alpha-hydroxycarboxylic acid, the carbohydrate, or the glycoside, more particularly a plant, a fungus, or bacteria, or constituents of plants, fungi, or bacteria, wood, more particularly in the form of wood flour or wood chips, paper, more particularly wastepaper, algae, cyanobacteria, or silage, or a mixture of at least two of the stated substances, or has formed from at least one of the stated substances or from the mixture.

4. The method of any of the preceding claims, wherein the catalyst is oxidized by blown introduction of oxygen or of a gas mixture containing oxygen, more particularly more than 10 percent by volume, into the solution, or by addition of an oxidizing agent, more particularly H₂O₂ or N₂O, to the solution.

5. The method of any of the preceding claims, wherein the solution comprises a polar solvent, more particularly water, formic acid, an ionic liquid, or mixtures of these solvents.

6. The method of any of the preceding claims, wherein an inorganic acid, more particularly sulfuric acid, phosphoric acid, hydrochloric acid, or carbonic acid, a carboxylic acid, having more particularly 2 to 20 C atoms, or a dicarboxylic acid, having more particularly 2 to 20 C atoms, is added to the solution.

7. The method of any of the preceding claims, wherein a surfactant or other additive, more particularly a sulfonic acid, more particularly methanesulfonic acid, trifluoromethanesulfonic acid, camphorsulfonic acid, toluenesulfonic acid, more particularly para-toluenesulfonic acid, chlorosulfonic acid, xylenesulfonic acid, benzenesulfonic acid, or a derivative of one of said acids, more particularly chlorobenzenesulfonic acid, more particularly para-chlorobenzenesulfonic acid, or a salt of one of said acids or of the derivative, or another substance which in an aqueous solution forms one of said acids or the derivative, is added to the solution.

8. The method of any of the preceding claims, wherein the contacting takes place at a temperature of 15 to 120°C, more particularly 50 to 110°C, more particularly 60 to 100°C, more particularly 70 to 95°C, more particularly 85 to 95°C.

9. The method of any of the preceding claims, wherein the contacting takes place under an oxygen partial pressure of 1 to 500 bar, more particularly 5 to 150 bar, more particularly 20 to 120 bar, more particularly 30 to 80 bar, more particularly 50 to 75 bar.

10. The method of any of the preceding claims, wherein the formic acid produced in the method is removed from the solution by distillation, reactive distillation, extraction, more particularly together with the catalyst, more particularly by reaction with a base, more particularly an amine, by stripping or, after reaction with a heterogeneous catalyst, more particularly platinum or palladium, it is removed from the solution in the form of reaction products - hydrogen and carbon dioxide - formed during the reaction.

11. The method of claim 10, wherein an ether, more particularly diisopropyl ether, ethyl tert-butyl ether, ethyl isobutyl ether, ethyl sec-butyl ether, methyl tert-pentyl ether, di-n-propyl ether, ethyl n-butyl ether, ethyl tert-pentyl ether, diisobutyl ether, or di-n-butyl ether, is added as extractant to the solution for the purpose of extracting the formic acid, or an amide, more particularly N,N-di-n-butylformamide, N-di-n-acetamide, N-methyl-N-heptylformamide, N-n-butyl-N-2-ethylhexylformamide, or N-n-butyl-N-cyclohexylformamide, is added for the purpose of extracting the formic acid together with the catalyst.

## Revendications

1. Procédé de production catalytique d'acide formique, dans lequel un ion de polyoxométallate servant de catalyseur de la formule générale [PMoₓV_{y}O₄₀]⁵⁻ est amené en contact dans une solution liquide à une température inférieure à 120°C avec un alpha-hydroxyaldéhyde, un acide alpha-hydroxycarboxylique, un glucide ou un glycoside, dans lequel 6 < x < 11 et 1 < y < 6 et x + y = 12, dans lequel x et y sont à chaque fois un nombre entier.

2. Procédé selon la revendication 1, dans lequel le catalyseur réduit lors du procédé est de nouveau converti dans son état de départ par oxydation.

3. Procédé selon l'une quelconque des revendications précédentes, dans lequel l'alpha-hydroxyaldéhyde, l'acide alpha-hydroxycarboxylique, le glucide ou le glycoside comprend un monosaccharide, notamment un aldose, un disaccharide, un oligosaccharide ou un polysaccharide, de l'amidon, de la cellulose, de l'hémicellulose, du glucose, du saccharose, du xylose, du cellobiose, du xylane, un hétérooligosaccharide, un hétéropolysaccharide, de l'acide glycolique ou de l'acide lactique ou un résidu contenant l'alpha-hydroxyaldéhyde, l'acide alpha-hydroxycarboxylique, le glucide ou le glycoside ou, notamment de la matière première croissante, notamment une plante, un champignon ou des bactéries ou des constituants de plantes, champignons ou bactéries, du bois, notamment sous la forme de farine de bois ou de copeaux de bois, du papier, notamment des vieux papiers, des algues, des cyanobactéries ou du ensilage, ou un mélange d'au moins deux des substances citées ou est généré à partir d'au moins une des substances citées ou du mélange.

4. Procédé selon l'une quelconque des revendications précédentes, dans lequel l'oxydation du catalyseur s'effectue par injection d'oxygène ou d'un mélange gazeux contenant de l'oxygène, notamment plus de 10 pour cent en volume, dans la solution ou par addition d'un agent oxydant, notamment H₂O₂ ou N₂O, à la solution.

5. Procédé selon l'une quelconque des revendications précédentes, dans lequel la solution comprend un solvant polaire, notamment de l'eau, de l'acide formique, un liquide ionique ou des mélanges de ces solvants.

6. Procédé selon l'une quelconque des revendications précédentes, dans lequel un acide inorganique, notamment de l'acide sulfurique, de l'acide phosphorique, de l'acide chlorhydrique ou de l'acide carbonique, un acide carboxylique présentant notamment 2 à 20 atomes de C ou un acide dicarboxylique présentant notamment 2 à 20 atomes de C est ajouté à la solution.

7. Procédé selon l'une quelconque des revendications précédentes, dans lequel un tensioactif ou un autre additif, notamment un acide sulfonique, notamment l'acide méthanesulfonique, l'acide trifluorométhanesulfonique, l'acide camphosulfonique, l'acide toluènesulfonique, notamment l'acide para-toluènesulfonique, l'acide chlorosulfonique, l'acide xylènesulfonique, l'acide benzènesulfonique ou un dérivé d'un des acides cités, notamment l'acide chlorobenzènesulfonique, notamment l'acide para-chlorobenzènesulfonique, ou un sel d'un des acides cités ou du dérivé ou une autre substance qui forme un des acides cités ou le dérivé dans une solution aqueuse, est ajouté à la solution.

8. Procédé selon l'une quelconque des revendications précédentes, dans lequel la mise en contact s'effectue à une température de 15 à 120°C, notamment 50 à 110°C, notamment 60 à 100°C, notamment 70 à 95°C, notamment 85 à 95°C.

9. Procédé selon l'une quelconque des revendications précédentes, dans lequel la mise en contact s'effectue sous une pression partielle d'oxygène de 1 à 500 bars, notamment 5 à 150 bars, notamment 20 à 120 bars, notamment 30 à 80 bars, notamment 50 à 75 bars.

10. Procédé selon l'une quelconque des revendications précédentes, dans lequel l'acide formique produit lors du procédé est éliminé de la solution par distillation, distillation réactive, extraction, notamment avec le catalyseur, notamment par conversion avec une base, notamment une amine, par lavage ou après conversion avec un catalyseur hétérogène, notamment du platine ou du palladium, en tant que produits réactionnels générés ce faisant que sont l'hydrogène et le dioxyde de carbone.

11. Procédé selon la revendication 10, dans lequel un éther, notamment l'éther di-isopropylique, l'éther éthyl-tert.-butylique, l'éther éthyl-isobutylique, l'éther éthyl-sec.-butylique, l'éther méthyl-tert.-pentylique, l'éther di-n-propylique, l'éther éthyl-n-butylique, l'éther éthyl-tert.-pentylique, l'éther diisobutylique ou l'éther di-n-butylique est ajouté à la solution pour l'extraction de l'acide formique en tant qu'agent d'extraction, ou un amide, notamment le N,N-di-n-butylformamide, le N-di-n-acétamide, le N-méthyl-N-heptylformamide, le N-n-butyl-N-2-éthylhexylformamide ou le N-n-butyl-N-cyclohexylformamide est ajouté à la solution pour l'extraction de l'acide formique avec le catalyseur.
